# EUROPÄISCHE PATENTANMELDUNG

(11) **EP 4 660 179 A1**
(43) Veröffentlichungstag der Anmeldung: **10.12.2025**
(21) Anmeldenummer: 24179589.7
(22) Anmeldetag: 03.06.2024
(51) Int. Cl.: C07C 5/05, C07C 11/08, C07C 7/00, C07C 9/12, C07C 7/04, C07C 9/10, C07C 41/06, C07C 43/04, C07C 7/148, C10G 11/00

(54) **VERFAHREN ZUR HERSTELLUNG VON HOCHREINEM 1-BUTEN UND VON HOCHREINEM ISOBUTAN**

(71) Anmelder: Evonik Oxeno GmbH & Co. KG, 45772 Marl (DE)
(72) Erfinder: PEITZ, Dr., Stephan, 45739 Oer-Erkenschwick (DE); STOCHNIOL, Dr., Guido, 45721 Haltern am See (DE); FLEISCHER, Dr., Vinzenz, 45770 Marl (DE); MOELLER, Dr., Oliver, 45739 Oer-Erkenschwick (DE)
(74) Vertreter: Evonik Patent Association

(57) **Zusammenfassung**

Gegenstand der Erfindung ist ein Verfahren zur Herstellung von hochreinem 1-Buten und hochreinem Isobuten aus zwei C4-Kohlenwasserstoffströmen, bei dem die beiden Ströme zum Teil gemeinsam verarbeitet werden. Weiterhin betrifft die vorliegende Erfindung auch eine Vorrichtung zur Durchführung des erfindungsgemäßen Verfahrens.

## Beschreibung

Die vorliegende Erfindung betrifft ein Verfahren zur Herstellung von hochreinem 1-Buten und hochreinem Isobuten aus zwei C4-Kohlenwasserstoffströmen, bei dem die beiden Ströme zum Teil gemeinsam verarbeitet werden. Weiterhin betrifft die vorliegende Erfindung auch eine Vorrichtung zur Durchführung des erfindungsgemäßen Verfahrens.

Verfahren zur Herstellung von hochreinem 1-Buten und hochreinem Isobutan sind bekannt und in der Literatur beschrieben, beispielsweise in der WO 2021/071815 A1. Diese Verfahren zeichnen sich insbesondere dadurch aus, dass zunächst Isobuten und Butadien aus den eingesetzten C4-Kohlenwasserstoffströmen entfernt werden. Isobuten lässt sich beispielsweise durch Umsetzung zu MTBE oder Isobutendimeren und anschließende Abtrennung der gebildeten Produkte entfernen. Butadien kann mittels Extraktion und ggf. Selektivhydrierung entfernt werden. Die weitere Aufarbeitung unter Erhalt eines hochreinen 1-Buten- und eines hochreinen Isobutanstroms erfolgt zumeist destillativ.

Ein Nachteil an dem in der WO 2021/071815 A1 offenbarten Verfahren ist, dass dort zwei separat voneinander vorliegende Produktionsstränge betrieben werden müssen, um die beiden eingesetzten C4-Kohlenwasserstoffströme verarbeiten zu können. Damit gehen jedoch hohe Investitions- und Betriebskosten einher. Zudem werden für den Betrieb von zwei separaten Produktionssträngen hohe Mengen an Energie benötigt.

Die zugrundeliegende Aufgabe der vorliegenden Erfindung war deshalb die Bereitstellung eines Verfahrens und einer Vorrichtung, bei der diese Probleme nicht auftreten. Es sollen möglichst wenig parallele Produktionsanlagen parallel betrieben werden, um Investitionskosten und Energie zu sparen. Trotzdem sollen mit dem Verfahren ein hochreiner 1-Buten- und ein hochreiner Isobutanstrom erhalten werden können. Die Einsparungen dürfen also nicht zu Lasten der Reinheit des 1 -Buten- und des Isobutanstroms gehen.

Diese Aufgabe konnte durch das vorliegende Verfahren zur Herstellung von hochreinem 1-Buten und hochreinem Isobutan nach Anspruch 1 gelöst werden. Bevorzugte Ausgestaltungen sind in den abhängigen Ansprüchen angegeben.

Die Aufgabe konnte durch das erfindungsgemäße Verfahren nach Anspruch 1 gelöst werden. Bevorzugte Ausführungsformen sind in den abhängigen Ansprüchen angegeben. Das erfindungsgemäße Verfahren ist ein Verfahren zur Herstellung von hochreinem 1-Buten und hochreinem Isobutan, wobei das Verfahren die folgenden Schritte umfasst:
a) Bereitstellen eines ersten C4-Kohlenwasserstoffstroms A und eines zweiten C4-Kohlenwasserstoffstroms B, wobei die beiden Ströme A und B jeweils zumindest 1,3-Butadien, Isobuten, Isobutan, 1-Buten und 2-Buten enthalten und wobei die Konzentration an Isobutan im Strom A höher ist als im Strom B;
b) Zuführen des Stroms A zu einer Isobutanabtrennung, wobei zumindest ein Teil des im Strom A vorhandenen Isobutans abgetrennt wird und dadurch ein an Isobutan abgereicherter Strom entsteht;
c) Abtrennen eines Teils des an Isobutan abgereicherten Stroms und Vermischen dieses Teils mit dem Strom B unter Erhalt eines Stroms C;
d) Zuführen von Strom C und Zuführen eines Alkohols, vorzugsweise von Methanol oder Ethanol, besonders bevorzugt Methanol zu einer Reaktionseinheit, wobei zumindest ein Teil des in Strom C enthaltenen Isobutens zu ATBE (Alkyl-tert.-butyl-ether), vorzugsweise MTBE (Methyl-tert.-butyl-ether) oder ETBE (Ethyl-tert.-butyl-ether), besonders bevorzugt MTBE (Methyl-tert.-butyl-ether) und/oder zu Isobutendimeren umgesetzt und ein Reaktionsaustrag erhalten wird, wobei der Reaktionsaustrag einer Produktabtrennung unterworfen wird, bei der ein Reststrom, der zumindest Alkohol, vorzugsweise Methanol oder Ethanol, besonders bevorzugt Methanol, 1,3-Butadien, 1-Buten, 2-Buten und Isobutan enthält, und ein Produktstrom, der zumindest das ATBE, vorzugsweise MTBE oder ETBE, besonders bevorzugt MTBE und/oder die Isobutendimere enthält anfallen;
e) Zuführen des Reststroms zu einer ersten Abtrenneinheit, wobei in der ersten Abtrenneinheit ein Leichtsiederstrom, der zumindest 1,3-Butadien, 1-Buten, 2-Buten und Isobutan enthält, und ein Wasserhaltiger Strom, der zumindest Alkohol, vorzugsweise Methanol oder Ethanol, besonders bevorzugt Methanol und Wasser enthält, anfallen;
f) Zuführen des Wasserhaltigen Stroms zu einer Rückgewinnungseinheit, um den Alkohol, vorzugsweise Methanol und Ethanol, besonders bevorzugt Methanol zumindest teilweise aus dem Wasser abzutrennen und Rückführung zumindest eines Teils des so erhaltenen Alkohols, vorzugsweise Methanols oder Ethanols, besonders bevorzugt Methanols zur Reaktionseinheit;
g) Zuführen des Leichtsiederstroms zu einer Hydrierung, um zumindest einen Teil des enthaltenen 1,3-Butadiens zu hydrieren, wodurch ein hydrierter Leichtsiederstrom erhalten wird;
h) Zuführen des hydrierten Leichtsiederstroms zu einer zweiten Abtrenneinheit, wobei in der zweiten Abtrenneinheit ein Strom D, der zumindest Isobutan und 1-Buten enthält, und ein Strom E, der zumindest n-Butan und 2-Buten enthält, anfallen;
i) Zuführen von Strom D zu einer dritten Abtrenneinheit, wobei in der dritten Abtrenneinheit ein Rohisobutanstrom und ein Strom an hochreinem 1-Buten anfallen;
j) Zuführen des Rohisobutanstroms zu einer vierten Abtrenneinheit, wobei in der vierten Abtrenneinheit ein Abgasstrom und ein Strom an hochreinem Isobutan anfallen.

Das erfindungsgemäße Verfahren hat den Vorteil, dass die beiden C4-Kohlenwasserstoffströme größtenteils in einer Produktionsanlage behandelt werden.

Der erste Schritt a) des erfindungsgemäßen Verfahrens zur Herstellung von hochreinem 1-Buten und hochreinem Isobutan ist die Bereitstellung eines ersten C4-Kohlenwasserstoffstroms A und eines zweiten C4-Kohlenwasserstoffstroms B, wobei die beiden Ströme A und B jeweils zumindest 1,3-Butadien, Isobuten, Isobutan, 1-Buten und 2-Buten enthalten und wobei die Konzentration an Isobutan im Strom A höher ist als im Strom B.

In dem erfindungsgemäßen Verfahren können alle üblicherweise zur Verfügung stehenden C4-Kohlenwasserstoffgemische eingesetzt werden. Geeignete C4-Kohlenwasserstoffströme sind beispielsweise Leichtbenzinfraktionen aus Raffinerien, C4-Fraktionen aus Crackern (beispielsweise Steamcracker (auch: Crack-C4), Hydrocracker, Fluid-Katcracker (FCC-C4)), Gemische aus Fischer-Tropsch-Synthesen, Gemische aus der Dehydrierung von Butanen, Gemische aus Skelettisomerisierung linearer Butene und Gemische, entstanden durch Metathese von Olefinen. Diese Techniken sind in der Fachliteratur beschrieben.

Die eingesetzten C4-Kohlenwasserstoffströme A und B können grundsätzlich auf die gleiche Weise oder durch das gleiche Verfahren hergestellt werden, wobei die Ströme aber unterschiedliche Mengen an Isobutan aufweisen. Das Verfahren zielt jedoch insbesondere auf die gleichzeitige Produktion von hochreinen 1-Buten- und Isobutanströmen aus zwei unterschiedlich hergestellten bzw. erhaltenen C4-Kohlenwasserstoffströme. In einer besonders bevorzugten Ausführungsform der vorliegenden Erfindung ist Strom A ein FCC-C4-Strom, also ein C4-Kohlenwasserstoffstrom aus einem Fluid-Katcracker. Strom B ist besonders bevorzugt ein Crack-C4-Strom, also ein C4-Kohlenwasserstoffstrom aus einem Steamcracker, bzw. ein Raffinat I.

Durch diese vorgelagerten Verfahren zur Produktion von C4-Kohlenwasserstoffströmen werden zwar ähnliche chemische Verbindungen produziert, führen jedoch zu Strömen mit unterschiedlicher Zusammensetzung der vorhandenen C4-Verbindungen. Die in dem erfindungsgemäßen Verfahren eingesetzten C4-Kohlenwasserstoffströme weisen bevorzugt die folgenden Zusammensetzungen auf:

**Tabelle 1: Typische Zusammensetzung von Crack-C4, Raffinat I und FCC-C4**

| Komponente | Crack-C4* | Raffinat I* | FCC-C4* |
|---|---|---|---|
| | Massen-% | Massen-% | Massen-% |
| Isobutan | 0,6 - 6 | 2 - 5 | 20 - 40 |
| n-Butan | 0,5 - 11 | 7 - 12 | 5 - 15 |
| 1-Buten | 9 - 25 | 25 - 31 | 10 - 20 |
| Isobuten | 10 - 35 | 40 - 48 | 10 - 20 |
| 2-Butene (cis und trans) | 4 - 20 | 11 - 15 | 20 - 35 |
| 1,3-Butadien | 25 - 70 | < 1 | < 1 |
| Weitere Komponenten (z. B. C3- oder C5-Verbindungen) | < 3 | < 1 | < 5 |

| | | | |
|---|---|---|---|
| * = Die Summe aller Komponenten ergibt 100 Massen-% | | | |

Die C4-Kohlenwasserstoffströme enthalten demnach je nach Crack-Verfahren unterschiedliche Mengen an Isobuten. Weitere Hauptbestandteile sind 1,3-Butadien, 1-Buten, 2-Buten (cis und trans), n-Butan und Isobutan. Typische Isobuten-Gehalte in der C4-Fraktion liegen bei Crack-C4 bei 10 bis 35 Massen-%, bei FCC-C4 bei 10 bis 20 Massen-%.

Für das erfindungsgemäße Verfahren ist es vorteilhaft, mehrfach ungesättigte Kohlenwasserstoffe wie 1,3-Butadien größtenteils aus dem Einsatzgemisch zu entfernen. Dadurch entsteht ein Raffinat I. Soll also Crack-C4 im erfindungsgemäßen Verfahren eingesetzt werden, muss vor dem Schritt a) 1,3-Butadien zumindest teilweise entfernt werden. Dies kann nach bekannten Verfahren, beispielsweise durch Extraktion, Extraktivdestillation oder Komplexbildung erfolgen. Eine Alternative zur Abtrennung der mehrfach ungesättigten Kohlenwasserstoffe ist eine selektive chemische Umsetzung. So kann beispielsweise 1,3-Butadien selektiv zu linearen Butenen hydriert werden, wie z. B. beschrieben in EP 0 523 482. Auch durch selektive Umsetzungen des 1,3-Butadiens, zum Beispiel Dimerisierung zum Cyclooctadien, Trimerisierung zum Cyclododecadien, Polymerisations- oder Telomerisationsreaktionen, kann das 1,3-Butadien zumindest teilweise entfernt werden.

### Schritt b)

Der in Schritt a) bereitgestellte C4-Kohlenwasserstoffstrom A wird in Schritt b) zu einer Isobutanabtrennung , wobei zumindest ein Teil des im Strom A vorhandenen Isobutans abgetrennt wird und dadurch ein an Isobutan abgereicherter Strom entsteht.

Die Konzentration des Isobutans im Strom Ab wird vorzugsweise mittels eines destillativen Schrittes in einer Destillation auf einen Wert von kleiner 5 Gew.-% gesenkt wird. Gleichzeitig werden auch die im Gemisch vorhandenen Leichtsieder (zum Beispiel C3-Kohlenwasserstoffe, leichte Sauerstoff, Stickstoff- und Schwefel enthaltende 5 Verbindungen) zumindest teilweise entfernt.

In einer bevorzugten Ausführungsformen kann der an Isobutan abgereicherter Strom aus Schritt b) einer Schwersiederabtrennung und/oder einer Abtrennung von Stickstoffhaltigen und/oder Schwefelhaltigen und/oder Sauerstoffhaltigen Verunreinigungen zugeführt werden, bevor der an Isobutan abgereicherte Strom zu Schritt c) geführt wird.

### Schwersiederabtrennung

Die Schwersiederabtrennung erfolgt vorzugsweise mittels Destillation. Schwersieder meint im vorliegenden Fall beispielsweise C5-Kohlenwasserstoffe. Mit der Schwersiederabtrennung können weiterhin Thioether abgetrennt werden. Die Thioether können durch Thioveretherung von Mercaptanen gebildet werden. Die Thioveretherung wird zur Entfernung der Mercaptane eingesetzt. Ein solches Verfahren ist beispielsweise in der WO 2014/009148 A1 offenbart.

Die destillative Abtrennung der Schwersieder wie C5-Kohlenwasserstoffe und ggf. den Thioethern erfolgt in mindestens einer Destillationskolonne. Die Schwersieder fallen dabei im Sumpf an. Der der an Isobutan abgereicherter Strom fällt demzufolge am Kopf der Destillationskolonne an.

Eine in diesem Verfahrensschritt bevorzugt eingesetzte Destillationskolonne weist vorzugsweise 40 bis 150 theoretische Trennstufen, bevorzugt 40 bis 100 und besonders bevorzugt 50 bis 80 theoretische Trennstufen auf. Das Rücklaufverhältnis beträgt, in Abhängigkeit von der realisierten Stufenzahl, der Zusammensetzung des Kolonnenzulaufes und der erforderlichen Reinheiten von Destillat und Sumpfprodukt, vorzugsweise zwischen 0,5 und 5, besonders bevorzugt zwischen 1 und 2,5. Das Rücklaufverhältnis ist hierbei definiert als Massenstrom des Rücklaufs geteilt durch den Massenstrom des Destillats. Die Kolonne wird vorzugsweise mit einem Betriebsdruck von 0,1 bis 2,0 MPa (absolut), bevorzugt von 0,5 bis 1,2 MPa (absolut) betrieben. Zur Beheizung der Kolonne kann z. B. Dampf eingesetzt werden. Die Kondensation kann, je nach gewähltem Betriebsdruck, gegen Kühlsole, Kühlwasser oder Luft erfolgen. Der Kopfbrüden der Kolonne kann aber auch mit anderen Kolonnen im Verfahren, z.B. mit der Kolonne zur Abtrennung des Isobutans, wärmeintegriert werden. In diesem Fall dient der Kondensator der Kolonne gleichzeitig als Verdampfer der Leichtsiederkolonne. Das Sumpfprodukt kann thermisch verwertet werden oder als Einsatzstoff anderer Prozesse, beispielsweise in einer Synthesegasanlage, genutzt werden

Für die Abtrennung von Stickstoffhaltigen und/oder Schwefelhaltigen und/oder Sauerstoffhaltigen Verunreinigungen können unterschiedliche Verfahren eingesetzt werden.

### Wasserwäsche

Durch eine Wasserwäsche können hydrophile Komponenten aus dem an Isobutan abgereicherten Strom ganz oder teilweise entfernt werden, beispielsweise Stickstoffkomponenten. Beispiele für Stickstoffkomponenten sind Acetonitril oder N-Methylpyrrolidon (die z. B. aus einer 1,3 Butadien-Extraktivdestillation stammen können). Auch Sauerstoffverbindungen (z. B. Aceton aus einer FCC-Einheit) können zum Teil über eine Wasserwäsche entfernt werden. Der an Isobutan abgereicherten Strom ist nach einer Wasserwäsche mit Wasser gesättigt. Um eine Zweiphasigkeit in den nachfolgenden Prozessschritten im Reaktor zu vermeiden, sollte dort die Reaktionstemperatur um ca. 10 °C über der Temperatur der Wasserwäsche liegen.

### Adsorptionsmittel

Adsorptionsmittel werden eingesetzt, um Verunreinigungen aus dem an Isobutan abgereicherten Strom zu entfernen. Dies kann beispielsweise vorteilhaft sein, wenn in einem der Prozessschritte Edelmetallkatalysatoren zum Einsatz kommen. Oftmals werden Stickstoff- oder Schwefelverbindungen über vorgeschaltete Adsorber entfernt. Beispiele für Adsorptionsmittel sind Aluminiumoxide, Molekularsiebe, Zeolithe, Aktivkohle, mit Metallen imprägnierte Tonerden. Adsorptionsmittel werden von diversen Firmen vertrieben, beispielsweise der Firma Alcoa (Selexsorb^{®}).

### Trocknung

In dem an Isobutan abgereicherten Strom gegebenenfalls enthaltenes Wasser, das beispielsweise aus der Wasserwäsche stammen kann, kann durch bekannte Verfahren zur Trocknung entfernt werden. Geeignete Verfahren sind beispielsweise die destillative Abtrennung des Wassers als Azeotrop. Dabei kann oftmals ein Azeotrop mit enthaltenen C4 Kohlenwasserstoffen ausgenutzt werden oder es können Schleppmittel zugesetzt werden.

### Schritt c)

In Schritt c) wird ein Teil des an Isobutan abgereicherten Stroms, der aus dem Schritt b) erhalten wird, abgetrennt. Der an Isobutan abgereicherten Strom wird folglich aufgetrennt. Die Abtrennung zumindest eines Teils des an Isobutan abgereicherten Stroms bzw. Auftrennung des Stroms in Schritt c) kann beispielsweise über ein Ventil mit Mengenregelung erfolgen. Entsprechende Ein- und Aufbauten sind dem Fachmann geläufig.

Der abgetrennte Teil des an Isobutan abgereicherten Stroms wird anschließend mit dem Strom B unter Erhalt eines Stroms C vermischt, der zu weiteren Prozessierung zu Schritt c) geführt wird.

Der andere Teil des an Isobutan abgereicherten Stroms wird unabhängig vom Strom C prozessiert und kann beispielsweise einer separaten Isobutenumsetzung zur Bildung von ATBE, vorzugsweise zur Bildung von MTBE oder zur Bildung ETBE, oder zur Bildung von Isobutendimeren zugeführt werden.

### Schritt d)

Nach dem Vermischen der beiden Ströme zu Strom C in Schritt c) werden Strom C und ein Alkohol, vorzugsweise Methanol oder Ethanol, besonders bevorzugt Methanol zu einer Reaktionseinheit geführt, wobei zumindest ein Teil des in Strom C enthaltenen Isobutens zu ATBE (Alkyl-tert.-butyl-ether), vorzugsweise MTBE (Methyl-tert.-butyl-ether) oder ETBE (Ethyl-tert.-butyl-ether), besonders bevorzugt MTBE (Methyl-tert.-butyl-ether) und/oder zu Isobutendimeren umgesetzt und ein Reaktionsaustrag erhalten wird. Der Reaktionsaustrag wird einer Produktabtrennung unterworfen, bei der ein Reststrom, der zumindest der zumindest Alkohol, vorzugsweise Methanol oder Ethanol, besonders bevorzugt Methanol, 1,3-Butadien, 1-Buten, 2-Buten und Isobutan enthält, und ein Produktstrom, der zumindest das ATBE, vorzugsweise MTBE oder ETBE, besonders bevorzugt MTBE und/oder die Isobutendimere enthält, anfallen.

Die Umsetzung in Schritt d) erfolgt in einem oder mehreren Reaktoren, die für die jeweilige Umsetzung geeignet. Sofern mehrere Reaktoren vorliegen, können diese Reaktoren parallel oder in Reihe geschaltet vorliegen. Es können demnach unterschiedliche Konfigurationen vorliegenden. Beispielsweise kann die Umsetzung in einer Reaktorreihe in einem Festbettreaktor oder einer Reihe von Festbettreaktoren verarbeitet werden und kann eine Zwischentrennungsstufe umfassen, um einen Teil des Produkts (ATBE oder Isobutendimer) zu entfernen. In einigen Ausführungsformen kann ein vorgelagerter Reaktoraustrag einem Abschlussreaktor zugeführt werden, bei dem es sich um eine Reaktivdestillation handeln kann, wo eine gleichzeitige Reaktion mindestens eines Teils des verbleibenden Isobutens und eine Trennung von Dimer oder ATBE von den verbleibenden C4-Komponenten, einschließlich n-Butan, Isobutan, 1-Buten und 2-Buten, ermöglicht wird. Die Reaktivdestillation entspricht dann auch der Produktabtrennung.

In einer bevorzugten Ausführungsform der vorliegenden Erfindung wird die Umsetzung in Schritt d) in mindestens zwei Reaktionsstufen durchgeführt wird, wobei mindestens die letzte Reaktionsstufe als Reaktivdestillation durchgeführt wird. Der Umsatz von ATBE in diesem Schritt beträgt vorzugsweise über 70 %, besonders bevorzugt über 90%.

Der bei der Umsetzung in Schritt d) eingesetzte Alkohol, vorzugsweise Methanol oder Ethanol, besonders bevorzugt Methanol kann entweder als Reaktant (zur Herstellung von ATBE bzw. MTBE oder ETBE) oder als Moderator (zur selektiven Dimerisierung zu Isobutendimeren) fungieren. Die beiden Alternativen werden nachfolgend genauer erläutert.

### Herstellung von ATBE, vorzugsweise MTBE oder ETBE

Sofern in Schritt d) eine ATBE-Herstellung bzw. MTBE- oder ETBE-Herstellung erfolgt, wird die Herstellung von ATBE vorzugsweise in zwei Stufen durchgeführt. Die erste Stufe der ATBE-Synthese im erfindungsgemäßen Verfahrensschritt d) wird vorzugsweise in Festbettreaktoren durchgeführt, die zweite Stufe der Umsetzung erfolgt vorzugsweise in einer Reaktivdestillation. Die Reaktivdestillation stellt in diesem Zusammenhang gleichzeitig die Produktabtrennung dar. Die erste Stufe der ATBE-Synthese wird vorzugsweise in mindestens zwei, besonders bevorzugt drei Festbettreaktoren durchgeführt. Als Reaktoren, in denen der Alkohol, vorzugsweise Methanol oder Ethanol, besonders bevorzugt Methanol mit dem Isobuten bis nahe an das thermodynamische Gleichgewicht umgesetzt wird, können herkömmliche Festbettreaktoren (Rohrbündelreaktoren, adiabatische Festbettreaktoren, Kreislaufreaktoren) eingesetzt werden. Durch die zweistufige ATBE-Synthese können insbesondere Isobuten-Restkonzentrationen in im Reaktionsaustrag von kleiner 1000 Massen-ppm, bevorzugt 800 Massen-ppm und besonders bevorzugt kleiner 500 Massen-ppm, bezogen auf das C4-Gemisch im Destillat, erhalten werden.

In der ersten Stufe erfolgt der Umsatz des Isobutens vorzugsweise bis zur Einstellung des thermodynamischen Gleichgewichts aus ATBE, Alkohol, vorzugsweise Methanol oder Ethanol, besonders bevorzugt Methanol und Isobuten, wobei vorzugsweise ein Isobuten-Umsatz von größer 94 %, besonders bevorzugt größer 96 % erreicht wird. Die Reaktoren der ersten Stufe werden vorzugsweise bei einer Temperatur von 20 bis 110 °C, bevorzugt 25 bis 70 °C und einem Druck von 0,5 bis 5 MPa, vorzugsweise 0,7 bis 2 MPa betrieben.

Da das thermodynamische Gleichgewicht zwischen Alkohol/Isobuten und Ether bei tiefer Temperatur überwiegend auf der Seite des Ethers liegt, ist es bevorzugt, den ersten der Reaktoren bei höherer Temperatur zwecks hoher Reaktionsgeschwindigkeit als die Folgenden, in denen der Gleichgewichtslage ausgenutzt wird, zu betreiben.

Das molare Verhältnis von Alkohol zu Isobuten (Alkohol: Isobuten) im Zulauf zum ersten Reaktor der ersten Stufe liegt vorzugsweise im Bereich von 10 : 1 bis 1 : 1, besonders bevorzugt von 5 : 1 bis 1,1 : 1 und ganz besonders bevorzugt im Bereich von 1,8 : 1 bis 1,2 : 1.

Die zweite Stufe der ATBE-Synthese wird vorzugsweise in einer Reaktivdestillationskolonne durchgeführt. Neben der weiteren Umsetzung von Isobuten zu ATBE erfolgt in der Reaktivdestillation auch die Produktabtrennung in den Reststrom, der zumindest Alkohol, vorzugsweise Methanol oder Ethanol, besonders bevorzugt Methanol, 1,3-Butadien, 1-Buten, 2-Buten und Isobutan enthält, den Produktstrom, der zumindest das ATBE und/oder die Isobutendimere enthält. Der so abgetrennte Reststrom wird im erfindungsgemäßen Schritt e) weiterbehandelt.

Die zweite Stufe der ATBE-Synthese wird weiterhin bevorzugt in einer Reaktivdestillationskolonne, die in einem Druckbereich mit Überdruck von 0,5 bis 1,5 MPa, vorzugsweise 0,75 bis 1,0 MPa und bei einer Temperatur in der Reaktionszone von 50 °C bis 90 °C, vorzugsweise 55 bis 70 °C bei einem Rücklaufverhältnis zwischen 0,5 und 1,5, bevorzugt zwischen 0,7 und 0,9 betrieben wird, an einem sauren lonenaustauscherharz, durchgeführt. Als Rücklaufverhältnis bezeichnet man definitionsgemäß das Verhältnis des Rücklaufstromes in die Kolonne zum abgeführten Destillatstrom. Die Temperatur des Kolonnenzulaufs liegt unabhängig von dessen Zusammensetzung, dem Reaktionsdruck in der Kolonne und dem Durchsatz vorzugsweise zwischen 50 °C und 90 °C, vorzugsweise zwischen 60 °C und 75 °C.

Der Zulauf zur Reaktivdestillationskolonne kann oberhalb oder unterhalb, vorzugsweise unterhalb der Katalysatorzone erfolgen. Der Zulauf zur Reaktivdestillationskolonne erfolgt vorzugsweise unterhalb der reaktiven Packung, bevorzugt 3 bis 13, besonders bevorzugt 4 bis 10 theoretische Trennstufen unterhalb der reaktiven Packung.

Optional kann zusätzlicher Alkohol, vorzugsweise Methanol oder Ethanol, besonders bevorzugt Methanol in die zweite Stufe eingespeist werden. Dies kann zusammen mit dem Zulauf aus der ersten Stufe oder aber auch an einer Stelle oder an mehreren Stellen der Reaktivdestillationskolonne erfolgen, z. B. am Kolonnenkopf und/oder auf, zwischen und/oder unter dem Katalysatorbett.

Die Reaktivdestillationskolonne enthält vorzugsweise in der Verstärkersäule den Katalysator und unter- und oberhalb der Katalysatorpackung befinden sich vorzugsweise Trennböden oder Destillationspackungen. Der Katalysator kann entweder in einer Packung integriert sein, wie beispielsweise in KataMax^{®}-Packungen, KataPak^{®}-Packungen oder MultiPak^{®}-Packungen oder auf Formkörper aufpolymerisiert sein. Bevorzugt werden KataMax^{®}-Packungen eingesetzt.

Bevorzugt weist die Reaktivdestillationskolonne oberhalb der Katalysatorpackung einen Bereich rein destillativer Trennung auf. Bevorzugt weist die Zone oberhalb der Katalysatorpackung 5 bis 20, insbesondere 10 bis 15 Trennstufen auf. Die Trennzone unterhalb des Katalysators umfasst 12 bis 36, insbesondere 20 bis 30 Trennstufen. Die Katalysatorzone kann mit einer destillativen Wirkung von 1 bis 5 theoretischer Trennstufen pro Meter Packungshöhe abgeschätzt werden. Die Höhe der Katalysatorzone/Reaktivzone lässt sich in Abhängigkeit vom gewünschten Isobuten-Umsatz durch einfache Vorversuche ermitteln. Die Katalysatormenge wird vorzugsweise so groß gewählt, dass ein Isobuten-Umsatz von 75 bis 99 %, vorzugsweise von 85 bis 98 % und besonders bevorzugt von 95 bis 97 % bezogen auf den Isobutengehalt im Zulauf zur Reaktivdestillation erreicht wird.

Als Katalysatoren werden in der ATBE-Synthese in Schritt d) vorzugsweise feste saure lonenaustauscherharze, die Sulfonsäuregruppen aufweisen, eingesetzt. Geeignete lonenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von lonenaustauscherharzen mit Sulfonsäuregruppen verwendet. Die Harze können gelförmig, makroporös oder schwammförmig hergestellt werden. Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren können die lonenaustauscherharze in ihrer H-Form eingesetzt werden. Stark saure Harze des Styrol-Divinylbenzol-Typs werden u. a. unter folgenden Handelsnamen verkauft: Duolite^{®} C20, Duolite^{®} C26, Amberlyst^{®} 15, Amberlyst^{®} 35, Amberlite^{®} IR-120, Amberlite^{®} 200, Dowex^{®} 50, Lewatit^{®} SPC 118, Lewatit^{®} SPC 108, K2611, K2621, OC 1501. Vorzugsweise werden als lonenaustauscherharze die Typen Amberlyst^{®} 15, Amberlyst^{®} 35 oder Lewatit^{®} K2621 eingesetzt.

Der in der zweiten Stufe der ATBE-Synthese, vorzugsweise der Reaktivdestillationskolonne als Sumpfprodukt anfallende ATBE kann für verschiedene Zwecke genutzt werden. Da er nur äußerst geringe Mengen an Alkyl-sec.-butylether (ASBE) enthält, ist er für die Herstellung von hochreinem Isobuten durch seine Rückspaltung geeignet, da praktisch keine linearen Butene durch Rückspaltung des Alkyl-sec.-butylethers entstehen können. Aufgrund des geringen Gehalts an Nebenprodukten (ASBE und C8-Olefine) kann der derart gewonnene ATBE nach Abtrennung von den restlichen Alkoholen als Lösemittel in der Analytik oder bei organischen Synthesen verwendet werden. Weiterhin ist seine Nutzung als Komponente für Ottokraftstoffe möglich.

Vorzugsweise wird die ATBE-Synthese in Verfahrensschritt d) des erfindungsgemäßen Verfahrens so durchgeführt wird, dass in der zweiten Stufe ein Kopfprodukt, enthaltend Alkohol, vorzugsweise Methanol oder Ethanol, besonders bevorzugt Methanol und ein C4-Kohlenwasserstoffgemisch (1,3-Butadien, 1-Buten, 2-Buten und Isobutan) mit einem Isobuten-Gehalt von weniger als 1000 Massen-ppm, bezogen auf das C4-Kohlenwasserstoffgemisch, sowie ein Produktstrom als Sumpfprodukt, enthaltend ATBE erhalten wird.

### Herstellung von Isobutendimeren

In einer Alternative wird das Isobuten in Schritt d) des erfindungsgemäßen Verfahrens zu Isobutendimeren (Diisobuten) umgesetzt. Die Herstellung der Isobutendimere in Schritt d) des Isobutens kann prinzipiell homogen katalysiert, d. h. unter Verwendung von im Reaktionsgemisch löslichen Katalysatoren, oder heterogen katalysiert, d. h. unter Verwendung von im Reaktionsgemisch unlöslichen Katalysatoren durchgeführt werden. Die Herstellung der Isobutendimere in Schritt d) erfolgt bevorzugt an festen heterogenen Katalysatoren, die weiterhin bevorzugt im Festbett angeordnet sind, so dass eine aufwändige Katalysatorabtrennung entfällt.

Als Festkatalysatoren können saure Stoffe eingesetzt werden, die im Edukt/Produktgemisch unlöslich sind. Die meisten dieser Katalysatoren gehören einer der folgenden Gruppen an:
a) Mineralsäuren (z. B. Schwefelsäure oder Phosphorsäure) auf einem Trägermaterial (z. B. Aluminiumoxid oder Siliciumdioxid),
b) Zeolithe oder andere Alumosilikate mit oder ohne Dotierung weiterer Metalle, insbesondere mit Übergangsmetallen oder
c) Saure lonenaustauscherharze, insbesondere saure Kationenaustauscher.

### Wegen der höheren Selektivität für die Bildung von Isobutenoligomeren und wegen der geringeren

Bildung von Nebenprodukten werden bevorzugt saure lonenaustauscherharze als Katalysator verwendet. Geeignete lonenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomeren sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von lonenaustauscherharzen mit Sulfongruppen verwendet. Die Eigenschaften dieser Harze, insbesondere spezifische Oberfläche, Porosität, Stabilität, Quellung bzw.

Schrumpfung und Austauschkapazität, können durch den Herstellprozess variiert werden. Die Harze können gelförmig, makroporös oder schwammförmig hergestellt werden. Stark saure Harze des Styrol-Divinylbenzol-Typs werden u. a. unter folgenden Handelsnamen verkauft: CT 151 der Firma Purolite^{®}, Amberlyst^{®} 15, Amberlyst^{®} 35, Amberlite^{®} IR-120, Amberlite^{®} 200, Dowex^{®} M-31, K 2611, K 2431.

Das saure lonenaustauscherharz wird zweckmäßig auf eine Aktivität eingestellt, die zwar die Oligomerisierung des Isobutens ermöglicht, jedoch die Cooligomerisierung von Isobuten mit linearen Butenen, die Oligomerisierung der linearen Butene sowie die Isomerisierung der linearen Butene kaum katalysiert. Weiterhin wird die Wärmeentwicklung damit im Reaktor auf einen technisch gut beherrschbaren Wert eingestellt.

Die Einstellung der gewünschten Katalysatoraktivität kann mit Hilfe von Moderatoren geschehen. Diese Stoffe werden zusammen mit dem Edukt über den Katalysator geleitet. Als Moderator wird Alkohol, vorzugsweise Methanol oder Ethanol, besonders bevorzugt Ethanol als Reinstoff oder als Gemisch eingesetzt. Die Herstellung der Isobutendimere wird daher bevorzugt in Gegenwart dieser Moderatoren durchgeführt. Bei der Verwendung von Moderatoren werden vorzugsweise Molverhältnisse von 0,01 bis 5, bevorzugt 0,01 bis 1, insbesondere 0,01 bis 0,7 Mol Moderator pro Mol Isobuten eingestellt.

Ein Reaktor in dem erfindungsgemäßen Verfahren kann ein Gemisch von lonenaustauscherharzen unterschiedlicher Reaktivität enthalten. Ebenso ist es möglich, dass ein Reaktor Katalysatoren mit unterschiedlicher Aktivität, z. B. in Schichten angeordnet, enthält. Wird mehr als ein Reaktor verwendet, können die einzelnen Reaktoren mit Katalysatoren gleicher oder unterschiedlicher Aktivität gefüllt sein.

Die im technischen Prozess eingesetzten Reaktoren können adiabatisch, polytrop oder praktisch isotherm betrieben werden. Praktisch isotherm bedeutet, dass die Temperatur an einer beliebigen Stelle im Reaktor maximal um 10 °C höher ist als die Temperatur am Reaktoreingang. Bei adiabatischem Betrieb der Reaktoren ist es in der Regel sinnvoll, mehrere Reaktoren in Reihe zu schalten und vorzugsweise zwischen den Reaktoren zu kühlen. Reaktoren, die für einen polytropen oder praktisch isothermen Betrieb geeignet sind, sind beispielsweise Rohrbündelreaktoren, wassergekühlte Rohrreaktoren (Kühlsystem auf Mantelseite), Rührkessel und Schlaufenreaktoren. Es ist möglich, mehrere Reaktoren, auch verschiedene Bauarten, zu kombinieren. Es ist zudem möglich, Reaktoren unter Rückführung von Produkt zu betreiben. In einer bevorzugten Ausführungsform der vorliegenden Erfindung wird die Herstellung von Isobutendimeren in mindestens zwei hintereinander geschalteten Reaktoren durchgeführt, wobei zwischen den Reaktoren eine Zwischenabtrennung der Dimere vorhanden ist.

Die Temperaturen bei der Herstellung der Isobutendimere in Schritt d) liegen vorzugsweise im Bereich von 15 bis 160 °C, vorzugsweise im Bereich von 40 bis 110 °C betragen.

Die Umsetzung kann mit und ohne Zugabe eines zusätzlichen Lösungsmittels erfolgen. Als Lösungsmittel werden bevorzugt gesättigte Kohlenwasserstoffe eingesetzt, insbesondere C4-, C8- oder C12-Kohlenwasserstoffe. Bei Zugabe von Lösungsmitteln beträgt ihr Anteil 0 bis 60 Massen-%, bevorzugt 0 bis 30 Massen %.

Die erfindungsgemäße Umsetzung kann bei einem Druck gleich oder über dem Dampfdruck von Strom C bei der jeweiligen Reaktionstemperatur durchgeführt werden, vorzugsweise bei einem Druck unter 40 bar, d. h. Strom C würde während der Dimerisierung ganz oder teilweise in flüssiger Phase vorliegen. Wenn die Reaktion vollständig in der Flüssigphase durchgeführt werden soll, sollte der Druck 2 bis 4 bar vorzugsweise höher als der Dampfdruck des Reaktionsgemisches sein, um in den Reaktoren Verdampfungsprobleme zu vermeiden.

Auch wenn die Reaktion bei einem Druck betrieben wird, bei dem die Reaktionsmischung nicht vollständig flüssig vorliegt (beispielsweise in einer Reaktivdestillation), findet die Oligomerisierung nach dem erfindungsgemäßen Verfahren trotzdem in der Flüssigphase, also an "feuchtem" d. h. mit Flüssigkeit benetztem Katalysator statt.

Der Gesamtumsatz an Isobuten zu Dimeren kann über die Art und Menge des verwendeten Katalysators, die eingestellten Reaktionsbedingungen und Anzahl der Reaktoren eingestellt werden. Im erfindungsgemäßen Verfahren werden vorzugsweise 30 bis 95 %, bevorzugt 50 bis 80 %, besonders bevorzugt 55 bis 70 %, des im Edukt enthaltenden Isobutens umgesetzt.

Das Reaktionsgemisch der Dimerisierung kann unterschiedlich aufgearbeitet werden. Die Produktabtrennung erfolgt vorzugsweise durch Destillation. Auch der Einsatz einer Reaktivdestillation ist möglich. Durch die Produktabtrennung werden ein Reststrom, der zumindest Methanol, 1,3-Butadien, 1-Buten, 2-Buten und Isobutan enthält, und ein Produktstrom, der zumindest die Isobutendimere enthält, erhalten.

Bevorzugt wird die Destillation bei einem Druck von 1 bis 10 bara (bara = bar absolut), besonders bevorzugt bei einem Druck von 4 bis 7 bara betrieben. Die Temperaturen im Sumpf betragen vorzugsweise von 120 bis 220 °C, besonders bevorzugt von 170 bis 200 °C. Das Rücklaufverhältnis wird vorzugsweise auf Werte von 0,1 bis 1,5, vorzugsweise von 0,3 bis 1,0 eingestellt. Die Destillation wird vorzugsweise in einer Kolonne mit einer Anzahl an Böden im Bereich von 20 bis 40, bevorzugt im Bereich von 25 bis 35 durchgeführt. Der so abgetrennte Reststrom wird im erfindungsgemäßen Schritt e) weiterbehandelt.

Der abgetrennte Produktstrom enthält hauptsächlich Isobutendimere (C8-Kohlenwasserstoffe) und kann gegebenenfalls einen Teil des eingesetzten Moderators aufweisen. Sie kann neben dem Diisobuten auch Codimere und höhere Oligomere (C12, C16+, etc.) enthalten. Der Anteil an Cooligomeren liegt bevorzugt unter 25 Massen-%. Der Produktstrom kann in weiteren Destillationsschritten aufgetrennt werden. So ist es beispielsweise möglich, eine Fraktion mit hochreinem Diisobuten abzutrennen, um diese separat, beispielsweise für chemische Synthesen, einzusetzen. Für den Einsatz als Kraftstoffkomponente für Ottomotoren kann es notwendig sein, hochsiedende Komponenten (vorzugsweise Siedepunkt > 220 °C) abzutrennen.

Es ist auch möglich die Oligomere bzw. Dimere ganz oder teilweise zu hydrieren. Methoden zur Hydrierung der Produkte der Oligomerisierung zu den entsprechenden Paraffinen sind dem Fachmann hinlänglich bekannt. In einer bevorzugten Ausführungsform wird die Hydrierung in flüssiger Phase an einem festen, im Hydriergut nicht löslichen Katalysator durchgeführt. Als Hydrierkatalysatoren werden bevorzugt Trägerkatalysatoren, die aus einem anorganischen Träger bestehen und als Aktivmetall Platin und/oder Palladium und/oder Nickel enthalten, verwendet. Die Temperatur, bei der die Hydrierung durchgeführt wird, liegt bevorzugt im Bereich von 10 bis 250 °C und der Druck zwischen 1 und 100 bar.

Nach der Hydrierung können durch destillative Trennung weitere Fraktionen gewonnen werden. Aus diesen und aus den unhydrierten Fraktionen sind durch Abmischung Kraftstoffadditive bestimmter Eigenschaften erhältlich. Weiterhin können einige Fraktionen als Lösemittel verwendet werden.

### Schritt e)

Im sich anschließenden Schritt e) wird der Reststrom aus Schritt d) zu einer ersten Abtrenneinheit geführt, wobei in der ersten Abtrenneinheit ein Leichtsiederstrom, der zumindest 1,3-Butadien, 1-Buten, 2-Buten und Isobutan enthält und ein Wasserhaltiger Strom, der zumindest den Alkohol, vorzugsweise Methanol oder Ethanol, besonders bevorzugt das Methanol und Wasser enthält, anfallen. Schritt e) betrifft demnach insbesondere eine Abtrennung des Alkohols, vorzugsweise von Methanol oder Ethanol, besonders bevorzugt von Methanol von den C4-Kohlenwasserstoffen.

Die Abtrennung des Alkohols, vorzugsweise des Methanols oder Ethanols, besonders bevorzugt des Methanols aus dem Reststrom erfolgt insbesondere durch Extraktion mit Wasser oder einer wässrigen Lösung als Waschmedium erfolgen. Der Alkohol, vorzugsweise das Methanol oder Ethanol wird also vorzugsweise in einem Extraktionsschritt mit Wasser oder einer wässrigen Lösung aus dem Reststrom ausgewaschen. Bevorzugt wird eine wässrige Lösung mit einem pH-Wert von größer-gleich 8, bevorzugt von 8 bis 12 eingesetzt. Die Einstellung des pH-Werts kann z. B. durch Zugabe von Natronlauge und/oder Schwefelsäure erfolgen. Diese Extraktion nach den bekannten Standardverfahren der Technik, kann beispielsweise in einer Extraktionskolonne oder in einer Kaskade von Mixern und Trennbehältern erfolgen. Gegenüber den anderen Verfahren weist sie verschiedene Vorteile auf, beispielsweise geringes Investment und niedrige Betriebskosten.

Durch die Abtrennung werden ein Wasserhaltiger Strom, der zumindest den Alkohol, vorzugsweise Methanol oder Ethanol, besonders bevorzugt Methanol und Wasser enthält, und ein Leichtsiederstrom, der zumindest 1,3-Butadien, 1-Buten, 2-Buten und Isobutan enthält, erhalten. Der Restgehalt an Alkohol im Leichtsiederstrom beträgt bevorzugt weniger als 0,2 Massen-%, besonders bevorzugt weniger als 500 Massen-ppm, ganz besonders bevorzugt weniger als 50 Massen-ppm.

Die erste Abtrenneinheit für die Extraktion des Alkohols, vorzugsweise des Methanols oder Ethanols, besonders bevorzugt des Methanols umfasst vorzugsweise mindestens eine Extraktionskolonne. Die mindestens eine Extraktionskolonne weist bevorzugt von 2 bis 25, besonders bevorzugt von 5 bis 15 theoretische Trennstufen auf und wird bevorzugt bei Temperaturen von 10 bis 90 °C und Drücken von mindestens 0,1 MPa über dem Dampfdruck der C4-Kohlenwasserstoffe betrieben. Das MassenVerhältnis von Waschmedium zu zugeführtem Reststrom beträgt vorzugsweise von 1 : 5 bis 1 : 40.

Bevorzugt wird der Reststrom in eine Extraktionskolonne überführt, in die im Gegenstrom mit dem Extraktionsmittel über einen am Kopf befindlichen Zulauf eingespeist wird. Das beladene Extraktionsmittel ist der Wasserhaltige Strom und kann über den Ablauf am Sumpf der Extraktionskolonne entnommen werden.

Das mit Alkohol beladene Waschwasser aus der Extraktion, also der Wasserhaltige Strom, wird in Schritt f) aufgearbeitet und anschließend zumindest teilweise in die Extraktion zurückgeführt.

### Schritt f)

In Schritt f) wird der Wasserhaltige Strom zu einer Rückgewinnungseinheit geführt, um den Alkohol, vorzugsweise das Methanol oder Ethanol, besonders bevorzugt das Methanol zumindest teilweise vom Wasser abzutrennen. Zumindest ein Teil des so erhaltenen Alkohols, vorzugsweise des Methanols oder Ethanols, besonders bevorzugt des Methanols wird zur Reaktionseinheit in Schritt d) zurückgeführt.

Die Aufarbeitung des Wasserhaltigen Stroms in der Rückgewinnungseinheit kann beispielsweise durch eine Destillation erfolgen, bei der eine praktisch alkoholfreie Wasserfraktion im Sumpf und Methanol als Kopfprodukt erhalten werden. Die Destillation wird vorzugsweise bei Überdruck durchgeführt, beispielsweise im Bereich von 1,1 bis 1,5 barü. Die Temperatur im Sumpf beträgt vorzugsweise 110 bis 140 °C. Die Temperatur am Kopf beträgt 80 bis 95 °C. Die Trennung von Alkohol, vorzugsweise Methanol oder Ethanol und Wasser ist dem Fachmann jedoch grundsätzlich bekannt. Der Alkohol, vorzugsweise das Methanol oder Ethanol kann zurück in die ATBE-Synthese oder die Herstellung der Isobutendimere in Schritt d) gefahren werden. Die Wasserfraktion aus dem Sumpf kann zur erneuten Verwendung zur ersten Abtrenneinheit in Schritt e) zurückgeführt werden.

### Schritt g)

Der Leichtsiederstrom wird in Schritt g) zu einer Hydrierung geführt, um zumindest einen Teil des enthaltenen 1,3-Butadiens zu hydrieren, wodurch ein hydrierter Leichtsiederstrom erhalten wird. Falls Spuren von Butadien nicht schon vor dem Verfahrensschritt g) entfernt wurden, können sie so aus dem Reststrom durch Selektivhydrierung (SHP) entfernt werden.

Die Hydrierung in Schritt g) kann in flüssiger Phase an einem Palladium-haltigen Festbettkatalysator mit Wasserstoff unter Zusatz von Kohlenmonoxid als Moderator erfolgen. Wasserstoff und Kohlenmonoxid sind dabei im Kohlenwasserstoffgemisch vollständig gelöst. Als Wasserstoffmenge wird mindestens die zugegeben, die stöchiometrisch für die Hydrierung der mehrfach ungesättigten Verbindungen zu den einfachen Olefinen notwendig ist. Sie lässt sich aus der Zusammensetzung des zu hydrierenden Leichtsiederstroms berechnen.

Die auf die Masse des Leichtsiederstroms zu beziehende CO-Menge beträgt von mindestens 0,05 Massen-ppm bis 100 Massen-ppm. Mengen von über 20 ppm führen standardmäßig zu keiner weiteren Verbesserung der Hydrierergebnisse, so dass Mengen von 0,05 bis 10 Massen-ppm bevorzugt sind. Die im jeweiligen Prozess optimal zu dosierende Menge an CO kann leicht experimentell, wie in DE 31 43 647 beschrieben, ermittelt werden.

Der Katalysator weist 0,1 bis 2 Massen-% Palladium und/oder Platin auf einem Träger auf. Zu solchen Trägern gehören beispielsweise Aluminiumoxid, Silicagel, Alumosilikat und Aktivkohle. Pro Liter eingesetztem Katalysator wird vorzugsweise eine Kohlenwasserstoffmenge im Bereich von 5 bis 300 Litern durchgesetzt.

Die Temperatur, bei der die Hydrierung durchgeführt wird, beträgt von 0 bis 75 °C. Damit kein freies Wasser anfällt, wird die Hydrierung zweckmäßig bei höherer Temperatur als die Extraktion in Verfahrensschritt e) betrieben.

Der Verfahrensdruck muss hinreichend groß sein, um die Flüssigphase bei der gewählten Temperatur aufrecht zu erhalten und um eine ausreichende Menge an Wasserstoff und Kohlenmonoxid in Lösung zu bringen. Der Reaktionsdruck beträgt kleiner 20 MPa, vorzugsweise kleiner 6 MPa, bevorzugt kleiner 2 MPa. Ein typischer Reaktionsdruck beträgt 1,5 MPa.

Die Hydrierung kann ein- oder mehrstufig durchgeführt werden. Einstufig bedeutet, dass nur ein einziger Reaktor eingesetzt wird. Mehrstufig bedeutet dementsprechend, dass mehrere Reaktoren vorliegen. Die einstufige Ausführung ist aus Kostengründen bevorzugt. Alternativ wird die Hydrierung mehrstufig, vorzugsweise zweistufig, durchgeführt. Die Einspeisung von Wasserstoff erfolgt dabei vor jedem der Reaktoren, die von Kohlenmonoxid vorzugsweise in den ersten der Reaktoren. Die Reaktoren können unter Rückführung von Produkt betrieben werden. In einer bevorzugten Ausführungsform wird die Hydrierung des zumindest einen Teils des 1,3-Butadiens in mindestens zwei Reaktionsstufen durchgeführt, wobei mindestens die letzte Reaktionsstufe in Anwesenheit von 0,05 bis 100 Massen-ppm CO durchgeführt wird.

### Schritt h)

Der aus dem Schritt g) erhaltene hydrierte Leichtsiederstrom enthält zumindest n-Butan, Isobutan, 1-Buten und 2-Buten (cis und trans), jedoch - wenn überhaupt - nur geringe Mengen im ppm-Bereich an 1,3-Butadien und/oder Isobuten. Der hydrierte Leichtsiederstrom wird dann in Schritt h) zu einer zweiten Abtrenneinheit geführt, wobei in der zweiten Abtrenneinheit ein Strom D, der zumindest Isobutan und 1-Buten enthält, und ein Strom E, der zumindest n-Butan und 2-Buten enthält, anfallen. Die Auftrennung in die Ströme D und E erfolgt vorzugsweise destillativ.

Aus dem hydrierten Leichtsiederstrom, in der Literatur auch als Raffinat II bezeichnet, können Isobutan und 1-Buten destillativ, vollständig oder teilweise als Strom D abgetrennt werden. Der zusätzlich anfallende Strom E, der auch als Raffinat III bezeichnet wird, enthält üblicherweise hauptsächlich 2-Butene, n-Butan und gegebenenfalls einen Anteil an 1-Buten. Strom E wird aus dem vorliegenden Verfahren ausgeschleust und kann beispielsweise als Einsatzstoffgemisch für eine Oligomerisierung eingesetzt werden. Die destillative Trennung kann in für die Auftrennung solcher Kohlenwasserstoffgemische üblicherweise eingesetzten Apparaturen, z. B. Destillations- oder Fraktionierungskolonnen, erfolgen.

In einer bevorzugten Ausführungsform wird die destillative Auftrennung in einer Superfraktionierkolonne durchgeführt. Der Zulauf zu dieser Superfraktionierkolonne erfolgt vorzugsweise in der unteren Hälfte, bevorzugt im unteren Drittel der Superfraktionierkolonne. Wegen der engen Siedelage des zu trennenden Gemisches wird Verfahrensschritt h) vorzugsweise in einer Superfraktionierkolonne, die mehr als 100, bevorzugt mehr als 125, besonders bevorzugt mehr als 150 theoretische Trennstufen und ganz besonders bevorzugt 150 bis 200 theoretische Trennstufen aufweist, durchgeführt. Die Superfraktionierkolonne kann als Packungs- oder Bodenkolonne ausgeführt sein. Bevorzugt ist die Ausführung als Bodenkolonne.

Das Rücklaufverhältnis (Rücklaufmenge zu Destillatabnahme) in der Superfraktionierkolonne beträgt, in Abhängigkeit von der realisierten Stufenzahl und vom Betriebsdruck, vorzugsweise kleiner-gleich 20, bevorzugt kleiner 14, besonders bevorzugt kleiner 11. Die Kondensation kann gegen Kühlwasser oder Luft durchgeführt werden. Möglich wäre es die Kondensationsenergie beispielsweise mittels einer Brüdenverdichtung oder einer Wärmepumpe nutzbar zu machen. Der Destillatbehälter wird vorzugsweise als Flüssigkeit-Flüssigkeit-Abscheider ausgeführt. Dadurch kann gegebenenfalls im Zulaufstrom enthaltenes Wasser als zweite Phase im Destillatbehälter abgetrennt werden und es kann ein technisch wasserfreies Sumpfprodukt (Strom E) erhalten werden.

Die Trennung gemäß Verfahrensschritt h) wird vorzugsweise bei einem Druck von 0,4 bis 1,0 MPaabsolut, bevorzugt bei einem Druck von 0,5 bis 0,7 MPaabsolut durchgeführt. Die Temperatur, bei derdie Trennung durchgeführt wird, beträgt vorzugsweise 35 bis 80 °C, bevorzugt 40 bis 65 °C.

Kolonnen zur Trennung von Stoffgemischen weisen üblicherweise mindestens einen Verdampfer auf, über den die zur Bewältigung der Trennaufgabe notwendige Energie eingebracht wird. Zur Beheizung des Verdampfers der in Verfahrensschritt h) eingesetzten Superfraktionierkolonne kann ein üblicher Wärmeträger, wie z. B. Dampf oder Warmwasser sowie bevorzugt Abwärme aus anderen Prozessen eingesetzt werden. In letzterem Fall kann es vorteilhaft sein, die Kolonne mit mehr als einem Verdampfer auszustatten.

Die Superfraktionierkolonne wird bevorzugt als einfache Kolonne mit mindestens einem Verdampfer und mindestens einem Kondensator ausgerüstet. Wegen des hohen Energiebedarfs und der kleinen Temperaturdifferenz zwischen Sumpf und Kopf der Kolonne sind Energiesparschaltungen besonders bevorzugte Ausführungsformen. Exemplarisch sei hier auf die Methode der Brüdenverdichtung verwiesen. Eine weitere besonders bevorzugte Schaltung ist die Zweidruckschaltung (double effect distillation) in Integration mit einer zweiten Kolonne. Dabei wird eine der Kolonnen bei so hohem Druck gefahren, dass ihre Kondensationstemperatur zur Beheizung der anderen Kolonne ausreicht. Bei der wärmetechnischen Verschaltung von Kolonnen mit unterschiedlichen Trennaufgaben kann prinzipiell jede geeignete Kolonne aus dem erfindungsgemäßen Verfahren aber auch eine Kolonne, die außerhalb des erfindungsgemäßen Verfahrens am Anlagenstandort vorhanden ist, mit der Kolonne des Verfahrensschrittes h) verschaltet werden. Besonders bevorzugt ist die zweite Kolonne die mindestens eine Kolonne aus dem nachfolgenden Verfahrensschritt i). Dabei ist es weiterhin bevorzugt, wenn die Superfraktionierkolonne aus dem vorliegenden Schritt h) einen höheren Druck aufweist, da hier die einfachere Trennaufgabe bewältigt wird.

### Schritt i)

Strom D wird im drauffolgenden Schritt i) zu einer dritten Abtrenneinheit geführt, wobei in der dritten Abtrenneinheit ein Rohisobutanstrom und ein Strom an hochreinem 1-Buten anfallen. In diesem Schritt wird das 1-Buten vom Isobutan im Strom D getrennt. Die Trennung erfolgt vorzugsweise mittels Destillation.

Durch die Abtrennung in Schritt i) wird ein hochreiner 1-Butenstrom erhalten, der einen Anteil von 1-Buten von mindestens 99 Massen-% bezogen auf den gesamten 1-Butenstrom aufweist.

In einer bevorzugten Ausgestaltung weist der 1-Butenstrom eine Reinheit von über 99,2 Massen-%, weiterhin bevorzugt über 99,3 Masse-%, weiterhin bevorzugt über 99,4 Massen-% und besonders bevorzugt über 99,5 Massen.-% jeweils bezogen auf den gesamten 1-Butenstrom auf. Weiterhin bevorzugt enthält der 1-Butenstrom vorzugsweise weniger als 5000 Massen-ppm, bevorzugt weniger als 2000 Massen-ppm und besonders bevorzugt weniger als 1500 Massen-ppm Isobuten.

In einer bevorzugten Ausführungsform erfolgt die Abtrennung des 1-Butens in mindestens einer Destillationskolonne, in der als Sumpfprodukt sehr reines 1-Buten erhalten wird. Als Kopfprodukt wird ein Rohisobutanstrom erhalten, der gegebenenfalls Leichtsieder (beispielsweise C3-Kohlenwasserstoffe) enthält und zu Schritt j) geführt wird.

Bevorzugt wird die Abtrennung in Schritt i) in einer Superfraktionierkolonne durchgeführt. Der Zulauf zu dieser Superfraktionierkolonne erfolgt vorzugsweise in die obere Hälfte, bevorzugt in die untere Hälfte der oberen Hälfte der Kolonne. Wegen der engen Siedelage des zu trennenden Gemisches wird die Superfraktionierkolonne mit vorzugsweise mehr als 100, bevorzugt mehr als 125, besonders bevorzugt mehr als 150 und ganz besonders bevorzugt von 150 bis 200 theoretischen Trennstufen ausgeführt. Die Superfraktionierkolonne wird vorzugsweise als Packungs- oder Bodenkolonne ausgeführt, bevorzugt als Bodenkolonne. Das Rücklaufverhältnis (Rücklaufmenge zu Destillatabnahme) ist, in Abhängigkeit von der realisierten Stufenzahl und vom Betriebsdruck, vorzugsweise kleiner-gleich 100, bevorzugt kleiner 70, besonders bevorzugt kleiner 60. Ganz besonders bevorzugt beträgt das Rücklaufverhältnis von 30 bis 60. Die Kondensation kann gegen Kühlwasser oder Luft durchgeführt werden. Der Destillatbehälter wird vorzugsweise als flüssig-flüssig-Abscheider ausgeführt. Dadurch kann gegebenenfalls im Zulaufstrom enthaltenes Wasser als zweite Phase im Destillatbehälter abgetrennt werden und es kann ein technisch wasserfreies Sumpfprodukt erhalten werden.

Kolonnen zur Trennung von Stoffgemischen weisen üblicherweise mindestens einen Verdampfer auf, über den die zur Bewältigung der Trennaufgabe notwendige Energie eingebracht wird. Zur Beheizung des Verdampfers der Kolonne kann ein üblicher Wärmeträger, wie z. B. Dampf oder Warmwasser, sowie bevorzugt Abwärme aus anderen Prozessen eingesetzt werden. In letzterem Fall kann es vorteilhaft sein, die Kolonne mit mehr als einem Verdampfer auszustatten.

Die Superfraktionierkolonne wird bevorzugt als einfache Kolonne mit mindestens einem Verdampfer und mindestens einem Kondensator ausgerüstet. Wegen des hohen Energiebedarfs und der kleinen Temperaturdifferenz zwischen Sumpf und Kopf der Kolonne sind Energiesparschaltungen besonders bevorzugte Ausführungsformen. Exemplarisch sei hier auf die Methode der Brüdenverdichtung verwiesen. Eine weitere besonders bevorzugte Schaltung ist die Zweidruckschaltung (double effect distillation) in Integration mit einer zweiten Kolonne. Dabei wird eine der Kolonnen bei so hohem Druck gefahren, dass ihre Kondensationstemperatur zur Beheizung der anderen Kolonne ausreicht. Bei der wärmetechnischen Verschaltung von Kolonnen mit unterschiedlichen Trennaufgaben kann prinzipiell jede geeignete Kolonne aus dem erfindungsgemäßen Verfahren aber auch eine Kolonne, die außerhalb des erfindungsgemäßen Verfahrens am Anlagenstandort vorhanden ist, mit der erfindungsgemäßen Kolonne des Verfahrensschrittes f) verschaltet werden. Besonders bevorzugt ist die Superfraktionierkolonne in diesem Schritt i) die zweite Kolonne und die Kolonne von Schritt h) die erste Kolonne. Dabei wird eine der Kolonnen, vorzugsweise die erste Kolonne, bei so hohem Druck gefahren, dass ihre Kondensationstemperatur zur Beheizung der anderen Kolonne ausreicht.

Neben dieser bevorzugten Ausführungsform des erfindungsgemäßen Verfahrensschrittes i) ist es auch möglich, aus Strom D in einer ersten Destillationskolonne zunächst gegebenenfalls vorhandene Leichtsieder als Kopfprodukt abzutrennen, wobei im Sumpf der Kolonne eine Mischung erhalten wird, die hauptsächlich 1-Buten und Isobutan enthält. In einer zweiten Kolonne, die wie die oben beschriebene Superfraktionierkolonne ausgeführt sein kann, kann diese Sumpfmischung in 1-Buten, welches als Sumpfprodukt anfällt, und eine Isobutan-reiche Fraktion (Kopfprodukt), aufgetrennt werden.

Mit dem erfindungsgemäßen Verfahren hergestelltes, hochreines 1-Buten ist ein gefragtes Zwischenprodukt. Es kann beispielsweise als Comonomer bei der Herstellung von Polyethylen (LLDPE oder HDPE) sowie von Ethylen-Propylen-Mischpolymeren eingesetzt werden. Es findet weiterhin Einsatz als Alkylierungsmittel und ist Ausgangsstoff für die Herstellung von Butan-2-ol, Butenoxid, Valeraldehyd.

Eine weitere Verwendung des erfindungsgemäß hergestellten nahezu Isobuten-freien 1-Butens ist die Herstellung von n-Buten-Oligomeren, insbesondere nach dem Octol-Prozess.

In einer alternativen Ausführungsform kann nach Schritt i) eine Hydrierung erfolgen, um Spuren von Olefinen zu den entsprechenden Alkanen zu hydrieren. Diese Hydrierung von Spuren an Olefinen ist dem Fachmann bekannt.

### Schritt j)

Der aus Schritt i) abgetrennte Rohisobutanstrom wird zu einer vierten Abtrenneinheit geführt, wobei in der vierten Abtrenneinheit ein Abgasstrom und ein Strom an hochreinem Isobutan anfallen.

Das bei der Aufarbeitung gewonnene Isobutan hat vorzugsweise eine Reinheit von mindestens 95 Massen-% Isobutan, weiterhin bevorzugt eine Reinheit von über 95,2 Massen-%, weiterhin bevorzugt eine Reinheit von über 95,3 Massen-%, weiterhin bevorzugt eine Reinheit von über 95,4 Massen-% und besonders bevorzugt eine Reinheit von über 95,5 Massen-%, gekoppelt mit einem Gesamtbutananteil (n-+ iso-Butan) von mindestens 99,5 Massen-%, bevorzugt 99,6 Massen-%, besonders bevorzugt 99,7 Massen-%. Der hochreine Isobutanstrom enthält weiterhin bevorzugt weniger als 1000 Massen-ppm, besonders bevorzugt weniger als 200 Massen-ppm Olefine. Der hochreine Isobutanstrom enthält weiterhin bevorzugt weniger als 100 Massen-ppm, besonders bevorzugt weniger als 10 Massen-ppm Oxygenate, wie z.B. Dimethylether oder Methanol.

Die Destillation wird vorzugsweise bei Überdrück vorzugsweise im Bereich von 8 bis 12 barü durchgeführt. Die Temperatur im Sumpf der Destillationskolonne beträgt vorzugsweise 65 bis 85 °C. Am Kopf der Destillationskolonne liegt die Temperatur vorzugsweise im Bereich von 60 bis 80 °C.

Ein weiterer Aspekt der vorliegenden Erfindung ist die Bereitstellung einer Vorrichtung zum Durchführen eines erfindungsgemäßen Verfahrens zur Herstellung von hochreinem 1-Buten und hochreinem Isobutan, umfassend eine Isobutanabtrenneinheit (12), die mindestens eine Destillationskolonne, in der zumindest ein Teil des im Strom A vorhandenen Isobutans abgetrennt wird, umfasst; eine Reaktionseinheit (14), die mindestens einen Reaktor umfasst und in der zumindest ein Teil des in Strom C enthaltenen Isobutens zu MTBE und/oder zu Isobutendimeren; eine erste Abtrenneinheit (16), die mindestens eine Destillationskolonne umfasst und in der ein Leichtsiederstrom, der zumindest 1,3-Butadien, 1-Buten, 2-Buten und Isobutan enthält, abgetrennt wird; eine Rückgewinnungseinheit (17) zur Rückgewinnung von Methanol; eine Hydriereinheit (18), die mindestens einen Hydrierreaktor umfasst und in der zumindest einen Teil des im Leichtsiederstroms enthaltenen 1,3-Butadiens selektiv hydriert wird; eine zweite Abtrenneinheit (20), die mindestens eine Destillationskolonne umfasst und in der ein Strom D, der zumindest Isobutan und 1-Buten enthält, abgetrennt wird; eine dritte Abtrenneinheit (22), die mindestens eine Destillationskolonne umfasst und in der hochreines 1-Buten abgetrennt wird; und eine vierte Abtrenneinheit (24), die mindestens eine Destillationskolonne umfasst und in der hochreines Isobutan abgetrennt wird.

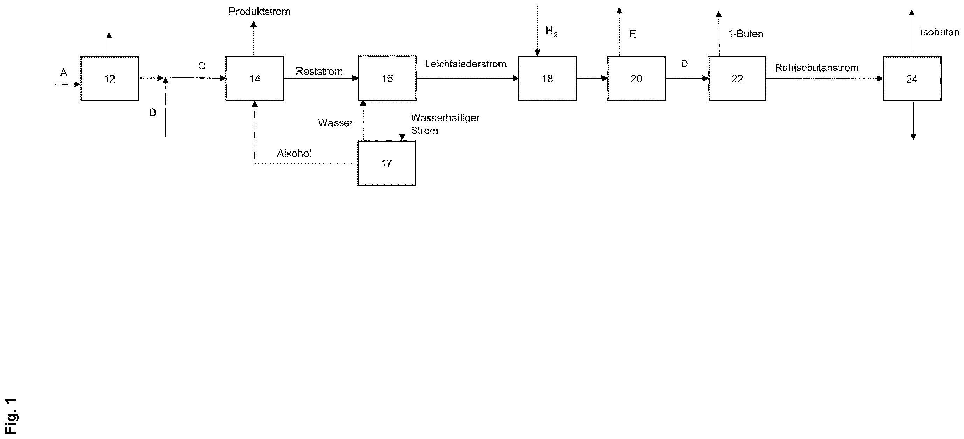

Die vorliegende Erfindung wird anhand des Fließschemas in Fig. 1 beschrieben. Die dort gezeigte Darstellung ist jedoch nur als Erläuterung und nicht einschränkend zu verstehen.

Fig. 1 zeigt ein Fließschema der vorliegenden Erfindung. Der erste C4-Kohlenwasserstoffstroms (A) wird zu einer Isobutanabtrennung (12) geführt, wobei zumindest ein Teil des im Strom A vorhandenen Isobutans abgetrennt wird und dadurch ein an Isobutan abgereicherter Strom entsteht. Der an Isobutan abgereicherten Stroms wird mit dem zweiten C4-Kohlenwasserstoffstrom (B) unter Erhalt eines Stroms (C) vermischt und zu einer Reaktionseinheit (14) geleitet. Das enthaltene Isobuten wird zumindest teilweise mit einem Alkohol, vorzugsweise mit Methanol oder Ethanol, besonders bevorzugt mit Methanol zu ATBE (Alkyl-tert.-butyl-ether), vorzugsweise MTBE (Methyl-tert.-butyl-ether) oder ETBE (Ethyl-tert.-butyl-ether), besonders bevorzugt MTBE (Methyl-tert.-butyl-ether) und/oder zu Isobutendimeren umgesetzt. In der Reaktionseinheit (14) befindet sich auch eine Produktabtrennung (nicht explizit gezeigt), bei der ein Reaktionsaustrag, wobei der Reaktionsaustrag einer Produktabtrennung unterworfen wird, bei der ein Reststrom, der zumindest Alkohol, vorzugsweise Methanol oder Ethanol, besonders bevorzugt Methanol, 1,3-Butadien, 1-Buten, 2-Buten und Isobutan enthält, und ein Produktstrom, der zumindest das MTBE und/oder die Isobutendimere enthält anfallen. Der Produktstrom wird ausgeschleust und der Reststrom wird zu einer ersten Abtrenneinheit (16) geführt, in der ein Leichtsiederstrom, der zumindest 1,3-Butadien, 1-Buten, 2-Buten und Isobutan enthält, und ein Wasserhaltiger Strom, der zumindest Alkohol, vorzugsweise Methanol oder Ethanol, besonders bevorzugt Methanol und Wasser enthält, anfallen. In der Rückgewinnungseinheit (17) wird der Alkohol, vorzugsweise Methanol und Ethanol, besonders bevorzugt Methanol zumindest teilweise aus dem Wasser abgetrennt Rückführung zumindest ein Teil des so erhaltenen Alkohols, vorzugsweise Methanols oder Ethanols, besonders bevorzugt Methanols zur Reaktionseinheit (14) zurückgeführt. Das Wasser kann zurück zur ersten Abtrenneinheit (16) zurückgeführt werden, was durch die gestrichelte Linie angedeutet wird. Der Leichtsiederstroms aus der ersten Abtrenneinheit (16) wird zu einer Hydrierung (18) geführt, wo 1,3-Butadien selektiv hydriert wird und ein hydrierter Leichtsiederstrom erhalten wird. Der hydrierte Leichtsiederstrom wird anschließend zu einer zweiten Abtrenneinheit (20) geleitet, wo ein Strom D, der zumindest Isobutan und 1-Buten enthält, und ein Strom E, der zumindest n-Butan und 2-Buten enthält, anfallen. Strom E wird ausgeschleust und Strom D zu einer dritten Abtrenneinheit (22) geführt, wo ein Rohisobutanstrom und ein Strom an hochreinem 1-Buten anfallen. Der Rohisobutanstroms wird in einer vierten Abtrenneinheit (24) aufgearbeitet, wobei ein Abgasstrom und ein Strom an hochreinem Isobutan anfallen.

## Patentansprüche

1. Verfahren zur Herstellung von hochreinem 1-Buten und hochreinem Isobutan, wobei das Verfahren die folgenden Schritte umfasst
a) Bereitstellen eines ersten C4-Kohlenwasserstoffstroms A und eines zweiten C4-Kohlenwasserstoffstroms B, wobei die beiden Ströme A und B jeweils zumindest 1,3-Butadien, Isobuten, Isobutan, 1-Buten und 2-Buten enthalten und wobei die Konzentration an Isobutan im Strom A höher ist als im Strom B;
b) Zuführen des Stroms A zu einer Isobutanabtrennung, wobei zumindest ein Teil des im Strom A vorhandenen Isobutans abgetrennt wird und dadurch ein an Isobutan abgereicherter Strom entsteht;
c) Abtrennen eines Teils des an Isobutan abgereicherten Stroms und Vermischen dieses Teils mit dem Strom B unter Erhalt eines Stroms C;
d) Zuführen von Strom C und Zuführen eines Alkohols, vorzugsweise von Methanol oder Ethanol, besonders bevorzugt Methanol zu einer Reaktionseinheit, wobei zumindest ein Teil des in Strom C enthaltenen Isobutens zu ATBE (Alkyl-tert.-butyl-ether), vorzugsweise MTBE (Methyl-tert.-butyl-ether) oder ETBE (Ethyl-tert.-butyl-ether), besonders bevorzugt MTBE (Methyl-tert.-butyl-ether) und/oder zu Isobutendimeren umgesetzt und ein Reaktionsaustrag erhalten wird, wobei der Reaktionsaustrag einer Produktabtrennung unterworfen wird, bei der ein Reststrom, der zumindest Alkohol, vorzugsweise Methanol oder Ethanol, besonders bevorzugt Methanol, 1,3-Butadien, 1-Buten, 2-Buten und Isobutan enthält, und ein Produktstrom, der zumindest das ATBE, vorzugsweise MTBE oder ETBE, besonders bevorzugt MTBE und/oder die Isobutendimere enthält anfallen;
e) Zuführen des Reststroms zu einer ersten Abtrenneinheit, wobei in der ersten Abtrenneinheit ein Leichtsiederstrom, der zumindest 1,3-Butadien, 1-Buten, 2-Buten und Isobutan enthält, und ein Wasserhaltiger Strom, der zumindest Alkohol, vorzugsweise Methanol oder Ethanol, besonders bevorzugt Methanol und Wasser enthält, anfallen;
f) Zuführen des Wasserhaltigen Stroms zu einer Rückgewinnungseinheit, um den Alkohol, vorzugsweise Methanol und Ethanol, besonders bevorzugt Methanol zumindest teilweise aus dem Wasser abzutrennen und Rückführung zumindest eines Teils des so erhaltenen Alkohols, vorzugsweise Methanols oder Ethanols, besonders bevorzugt Methanols zur Reaktionseinheit;
g) Zuführen des Leichtsiederstroms zu einer Hydrierung, um zumindest einen Teil des enthaltenen 1,3-Butadiens zu hydrieren, wodurch ein hydrierter Leichtsiederstrom erhalten wird;
h) Zuführen des hydrierten Leichtsiederstroms zu einer zweiten Abtrenneinheit, wobei in der zweiten Abtrenneinheit ein Strom D, der zumindest Isobutan und 1-Buten enthält, und ein Strom E, der zumindest n-Butan und 2-Buten enthält, anfallen;
i) Zuführen von Strom D zu einer dritten Abtrenneinheit, wobei in der dritten Abtrenneinheit ein Rohisobutanstrom und ein Strom an hochreinem 1-Buten anfallen;
j) Zuführen des Rohisobutanstroms zu einer vierten Abtrenneinheit, wobei in der vierten Abtrenneinheit ein Abgasstrom und ein Strom an hochreinem Isobutan anfallen.

2. Verfahren nach Anspruch 1, wobei der an Isobutan abgereichter Strom aus Schritt b) einer Schwersiederabtrennung und/oder einer Abtrennung von Stickstoffhaltigen und/oder Schwefelhaltigen und/oder Sauerstoffhaltigen Verunreinigungen zugeführt wird, bevor der an Isobutan abgereicherte Strom zu Schritt c) geführt wird.

3. Verfahren nach Anspruch 1 oder 2, wobei die Abtrennung zumindest eines Teils des an Isobutan abgereicherten Stroms in Schritt c) über ein Ventil mit Mengenregelung erfolgt.

4. Verfahren nach einem der vorhergehenden Ansprüche, wobei die Umsetzung in Schritt d) in mindestens zwei Reaktionsstufen durchgeführt wird, wobei mindestens die letzte Reaktionsstufe als Reaktivdestillation durchgeführt wird.

5. Verfahren nach einem der vorhergehenden Ansprüche, wobei in Schritt e) das Methanol in einem Extraktionsschritt mit Wasser oder einer wässrigen Lösung aus dem Reststrom ausgewaschen werden.

6. Verfahren nach einem der vorhergehenden Ansprüche, wobei die Hydrierung des zumindest einen Teils des 1,3-Butadiens in Schritt g) in mindestens zwei Reaktionsstufen erfolgt, wobei mindestens die letzte Reaktionsstufe in Anwesenheit von 0,05 bis 100 wppm CO durchgeführt wird.

7. Verfahren nach einem der vorhergehenden Ansprüche, wobei der Umsatz des Isobutens in Schritt d) über 70 %, vorzugsweise über 90% beträgt.

8. Verfahren nach einem der vorhergehenden Ansprüche, wobei bei der Umsetzung von Isobuten in Schritt d) als Katalysator ein lonentauscherharz eingesetzt wird.

9. Verfahren nach einem der vorhergehenden Ansprüche, wobei das in Schritt i) erhaltene hochreine 1-Buten eine Reinheit von mindestens 99 % aufweist.

10. Verfahren nach einem der vorhergehenden Ansprüche, wobei das in Schritt i) erhaltene hochreine 1-Buten weniger als 5000 Massen-ppm Isobuten enthält.

11. Verfahren nach einem der vorhergehenden Ansprüche, wobei das in Schritt j) erhaltene hochreine Isobutan eine Reinheit von mindestens 99 % aufweist.

12. Verfahren nach einem der vorhergehenden Ansprüche, wobei das in Schritt i) erhaltene hochreine 1-Buten weniger als 1000 Massen-ppm Olefine enthält.

13. Verfahren nach einem der vorhergehenden Ansprüche, wobei die erste Abtrenneinheit und/oder die dritte Abtrenneinheit und/oder die vierte Abtrenneinheit mindestens eine Destillationskolonne umfasst.

14. Vorrichtung zur Durchführung des Verfahrens nach einem der Ansprüche 1 bis 13, umfassend eine Isobutanabtrenneinheit (1), die mindestens eine Destillationskolonne, in der zumindest ein Teil des im Strom A vorhandenen Isobutans abgetrennt wird, umfasst; eine Reaktionseinheit (2), die mindestens einen Reaktor umfasst und in der zumindest ein Teil des in Strom C enthaltenen Isobutens zu MTBE und/oder zu Isobutendimeren; eine erste Abtrenneinheit (3), die mindestens eine Destillationskolonne umfasst und in der ein Leichtsiederstrom, der zumindest 1,3-Butadien, 1-Buten, 2-Buten und Isobutan enthält, abgetrennt wird; eine Rückgewinnungseinheit (4) zur Rückgewinnung von Methanol; eine Hydriereinheit (5), die mindestens einen Hydrierreaktor umfasst und in der zumindest einen Teil des im Leichtsiederstroms enthaltenen 1,3-Butadiens selektiv hydriert wird; eine zweite Abtrenneinheit (6), die mindestens eine Destillationskolonne umfasst und in der ein Strom D, der zumindest Isobutan und 1-Buten enthält, abgetrennt wird; eine dritte Abtrenneinheit (7), die mindestens eine Destillationskolonne umfasst und in der hochreines 1-Buten abgetrennt wird; und eine vierte Abtrenneinheit (8), die mindestens eine Destillationskolonne umfasst und in der hochreines Isobutan abgetrennt wird.
